# EUROPEAN PATENT APPLICATION

(11) **EP 3 808 364 A1**
(43) Date of publication of application: **21.04.2021**
(21) Application number: 19811507.3
(22) Date of filing: 30.05.2019
(51) Int. Cl.: A61K 38/16, A61K 38/17, A61P 25/00

(54) **METHOD AND COMPOSITION FOR TREATING HUNTER SYNDROME THROUGH CEREBRAL LATERAL VENTRICLE ADMINISTRATION**

(30) Priority: 30.05.2018 KR 20180061741
(71) Applicant: Medigenebio Corporation, Gangnam-gu Seoul 06164 (KR); Green Cross Corporation, Yongin-si, Gyeonggi-do 16924 (KR)
(72) Inventor: YEO, Jae Yeon, Seoul 06293 (KR)
(74) Representative: HGF
(86) International application number: PCT/KR2019/006515
(87) International publication number: WO 2019/231260

(57) **Abstract**

In one aspect, the present invention relates to a pharmaceutical composition comprising iduronate-2-sulfatase beta (IDS-β), which may be administered into the cerebral lateral ventricle once every four weeks to treat Hunter syndrome in a subject. Compared to a single administration of the same dose of an active substance, the present invention, due to repeated administrations over a long period of time, exhibits superior effects in treating Hunter syndrome, and further has the following effects which cannot be anticipated from the result of a single administration: treating or restoring a damaged brain structure; and substantially treating or improving brain functions, particularly, improving memory and learning.

<110>    MEDIGENEBIO CORPORATION

         GREEN CROSS CORPORATION

<120>    METHOD AND COMPOSITION FOR TREATING HUNTER SYNDROME BY
         ADMINISTRATION OF LATERAL VENTRICLE OF CEREBRUM

<130>    PCB905045GCC

<150>    KR 10-2018-0061741
<151>    2018-05-30

<160>    2

<170>    KoPatentIn 3.0

<210>    1
<211>    525
<212>    PRT
<213>    Artificial Sequence

<220>
<223>    recombinant human Iduronate-2-sulfatase protein


<400>    1
Ser Glu Thr Gln Ala Asn Ser Thr Thr Asp Ala Leu Asn Val Leu Leu
  1               5                  10                  15

Ile Ile Val Asp Asp Leu Arg Pro Ser Leu Gly Cys Tyr Gly Asp Lys
             20                  25                  30

Leu Val Arg Ser Pro Asn Ile Asp Gln Leu Ala Ser His Ser Leu Leu
         35                  40                  45

Phe Gln Asn Ala Phe Ala Gln Gln Ala Val Cys Ala Pro Ser Arg Val
     50                  55                  60

Ser Phe Leu Thr Gly Arg Arg Pro Asp Thr Thr Arg Leu Tyr Asp Phe
 65                  70                  75                  80

Asn Ser Tyr Trp Arg Val His Ala Gly Asn Phe Ser Thr Ile Pro Gln
                 85                  90                  95

Tyr Phe Lys Glu Asn Gly Tyr Val Thr Met Ser Val Gly Lys Val Phe
            100                 105                 110

His Pro Gly Ile Ser Ser Asn His Thr Asp Asp Ser Pro Tyr Ser Trp
        115                 120                 125

Ser Phe Pro Pro Tyr His Pro Ser Ser Glu Lys Tyr Glu Asn Thr Lys
    130                 135                 140

Thr Cys Arg Gly Pro Asp Gly Glu Leu His Ala Asn Leu Leu Cys Pro
145                 150                 155                 160

Val Asp Val Leu Asp Val Pro Glu Gly Thr Leu Pro Asp Lys Gln Ser
                165                 170                 175

Thr Glu Gln Ala Ile Gln Leu Leu Glu Lys Met Lys Thr Ser Ala Ser
            180                 185                 190

Pro Phe Phe Leu Ala Val Gly Tyr His Lys Pro His Ile Pro Phe Arg

195                 200                 205

Tyr Pro Lys Glu Phe Gln Lys Leu Tyr Pro Leu Glu Asn Ile Thr Leu
    210                 215                 220

Ala Pro Asp Pro Glu Val Pro Asp Gly Leu Pro Pro Val Ala Tyr Asn
225                 230                 235                 240

Pro Trp Met Asp Ile Arg Gln Arg Glu Asp Val Gln Ala Leu Asn Ile
                245                 250                 255

Ser Val Pro Tyr Gly Pro Ile Pro Val Asp Phe Gln Arg Lys Ile Arg
            260                 265                 270

Gln Ser Tyr Phe Ala Ser Val Ser Tyr Leu Asp Thr Gln Val Gly Arg
        275                 280                 285

Leu Leu Ser Ala Leu Asp Asp Leu Gln Leu Ala Asn Ser Thr Ile Ile
    290                 295                 300

Ala Phe Thr Ser Asp His Gly Trp Ala Leu Gly Glu His Gly Glu Trp
305                 310                 315                 320

Ala Lys Tyr Ser Asn Phe Asp Val Ala Thr His Val Pro Leu Ile Phe
                325                 330                 335

Tyr Val Pro Gly Arg Thr Ala Ser Leu Pro Glu Ala Gly Glu Lys Leu
            340                 345                 350

Phe Pro Tyr Leu Asp Pro Phe Asp Ser Ala Ser Gln Leu Met Glu Pro
        355                 360                 365

Gly Arg Gln Ser Met Asp Leu Val Glu Leu Val Ser Leu Phe Pro Thr
    370                 375                 380

Leu Ala Gly Leu Ala Gly Leu Gln Val Pro Pro Arg Cys Pro Val Pro
385                 390                 395                 400

Ser Phe His Val Glu Leu Cys Arg Glu Gly Lys Asn Leu Leu Lys His
                405                 410                 415

Phe Arg Phe Arg Asp Leu Glu Glu Asp Pro Tyr Leu Pro Gly Asn Pro
            420                 425                 430

Arg Glu Leu Ile Ala Tyr Ser Gln Tyr Pro Arg Pro Ser Asp Ile Pro
        435                 440                 445

Gln Trp Asn Ser Asp Lys Pro Ser Leu Lys Asp Ile Lys Ile Met Gly
    450                 455                 460

Tyr Ser Ile Arg Thr Ile Asp Tyr Arg Tyr Thr Val Trp Val Gly Phe
465                 470                 475                 480

Asn Pro Asp Glu Phe Leu Ala Asn Phe Ser Asp Ile His Ala Gly Glu
                485                 490                 495

Leu Tyr Phe Val Asp Ser Asp Pro Leu Gln Asp His Asn Met Tyr Asn
            500                 505                 510

Asp Ser Gln Gly Gly Asp Leu Phe Gln Leu Leu Met Pro
        515                 520                 525

<210>    2
<211>    525
<212>    PRT
<213>    Artificial Sequence

<220>
<223>    recombinant human Iduronate-2-sulfatase protein


<220>
<221>    MISC_FEATURE
<222>    (59)
<223>    The 59th amino acid "Gly" of SEQ ID NO:2 stands for a
         Formyl-Glycine


<400>    2
Ser Glu Thr Gln Ala Asn Ser Thr Thr Asp Ala Leu Asn Val Leu Leu
1               5                   10                  15

Ile Ile Val Asp Asp Leu Arg Pro Ser Leu Gly Cys Tyr Gly Asp Lys
            20                  25                  30

Leu Val Arg Ser Pro Asn Ile Asp Gln Leu Ala Ser His Ser Leu Leu
        35                  40                  45

Phe Gln Asn Ala Phe Ala Gln Gln Ala Val Gly Ala Pro Ser Arg Val
    50                  55                  60

Ser Phe Leu Thr Gly Arg Arg Pro Asp Thr Thr Arg Leu Tyr Asp Phe
65                  70                  75                  80

Asn Ser Tyr Trp Arg Val His Ala Gly Asn Phe Ser Thr Ile Pro Gln
                85                  90                  95

Tyr Phe Lys Glu Asn Gly Tyr Val Thr Met Ser Val Gly Lys Val Phe
            100                 105                 110

His Pro Gly Ile Ser Ser Asn His Thr Asp Asp Ser Pro Tyr Ser Trp
        115                 120                 125

Ser Phe Pro Pro Tyr His Pro Ser Ser Glu Lys Tyr Glu Asn Thr Lys
    130                 135                 140

Thr Cys Arg Gly Pro Asp Gly Glu Leu His Ala Asn Leu Leu Cys Pro
145                 150                 155                 160

Val Asp Val Leu Asp Val Pro Glu Gly Thr Leu Pro Asp Lys Gln Ser
                165                 170                 175

Thr Glu Gln Ala Ile Gln Leu Leu Glu Lys Met Lys Thr Ser Ala Ser
            180                 185                 190

Pro Phe Phe Leu Ala Val Gly Tyr His Lys Pro His Ile Pro Phe Arg
        195                 200                 205

Tyr Pro Lys Glu Phe Gln Lys Leu Tyr Pro Leu Glu Asn Ile Thr Leu
    210                 215                 220

Ala Pro Asp Pro Glu Val Pro Asp Gly Leu Pro Pro Val Ala Tyr Asn
225                 230                 235                 240

Pro Trp Met Asp Ile Arg Gln Arg Glu Asp Val Gln Ala Leu Asn Ile

245                 250                 255

Ser Val Pro Tyr Gly Pro Ile Pro Val Asp Phe Gln Arg Lys Ile Arg
            260                 265                 270

Gln Ser Tyr Phe Ala Ser Val Ser Tyr Leu Asp Thr Gln Val Gly Arg
        275                 280                 285

Leu Leu Ser Ala Leu Asp Asp Leu Gln Leu Ala Asn Ser Thr Ile Ile
    290                 295                 300

Ala Phe Thr Ser Asp His Gly Trp Ala Leu Gly Glu His Gly Glu Trp
305                 310                 315                 320

Ala Lys Tyr Ser Asn Phe Asp Val Ala Thr His Val Pro Leu Ile Phe
                325                 330                 335

Tyr Val Pro Gly Arg Thr Ala Ser Leu Pro Glu Ala Gly Glu Lys Leu
            340                 345                 350

Phe Pro Tyr Leu Asp Pro Phe Asp Ser Ala Ser Gln Leu Met Glu Pro
        355                 360                 365

Gly Arg Gln Ser Met Asp Leu Val Glu Leu Val Ser Leu Phe Pro Thr
    370                 375                 380

Leu Ala Gly Leu Ala Gly Leu Gln Val Pro Pro Arg Cys Pro Val Pro
385                 390                 395                 400

Ser Phe His Val Glu Leu Cys Arg Glu Gly Lys Asn Leu Leu Lys His
                405                 410                 415

Phe Arg Phe Arg Asp Leu Glu Glu Asp Pro Tyr Leu Pro Gly Asn Pro
            420                 425                 430

Arg Glu Leu Ile Ala Tyr Ser Gln Tyr Pro Arg Pro Ser Asp Ile Pro
        435                 440                 445

Gln Trp Asn Ser Asp Lys Pro Ser Leu Lys Asp Ile Lys Ile Met Gly
    450                 455                 460

Tyr Ser Ile Arg Thr Ile Asp Tyr Arg Tyr Thr Val Trp Val Gly Phe
465                 470                 475                 480

Asn Pro Asp Glu Phe Leu Ala Asn Phe Ser Asp Ile His Ala Gly Glu
                485                 490                 495

Leu Tyr Phe Val Asp Ser Asp Pro Leu Gln Asp His Asn Met Tyr Asn
            500                 505                 510

Asp Ser Gln Gly Gly Asp Leu Phe Gln Leu Leu Met Pro
        515                 520                 525

## Description

### Technical Field

The present invention relates to a method and a pharmaceutical composition for treating Hunter syndrome.

### Background Art

Hunter syndrome or mucosaccharidosis type II is one of lysosomal storage diseases (LSDs) in which mucopolysaccharides such as glycosaminoglycans (GAGs) are not degraded in the body due to deficiency of iduronate-2-sulfatase (IDS or I2S) and thus accumulated in lysosomes. GAGs are accumulated in cells throughout the body and cause various symptoms. The symptoms include prominent facial features, large head, abdominal distension caused by enlarged liver or spleen, and the like, which occur with hearing loss, heart valve disease, obstructive respiratory disease, sleep apnea, and the like. In addition, accumulated GAGs may also limit joint movement, and may cause neurological symptoms and delayed development when the central nervous system is affected. Hunter syndrome is known to occur in approximately 1 in 162,000 live births and is inherited in an X-linked recessive pattern.

Enzyme replacement therapy (ERT), which is one of the treatment methods for Hunter syndrome, is a method in which externally produced IDS is administered into the body. Such a method has an advantage of being simple in terms of administration while having a disadvantage of high treatment cost in that the enzyme needs to be continuously administrated. Elaprase® (Shire Pharmaceuticals Group), which is a recombinantly produced enzyme replacement therapy and has been approved by the US FDA, is on the market, and has disadvantages in that unit price thereof is very high and it exhibits low efficacy and safety.

The enzyme replacement therapy is achieved by systemic administration of naturally-occurring or recombinant-derived proteins and/or enzymes to a subject. Approved enzyme replacement therapies are typically administered intravenously and are generally effective in treating physical symptoms resulting from an enzyme deficiency that is responsible for a disease or condition. However, these enzyme replacement therapies are problematic in that the intravenously administered proteins and/or enzymes are limitedly distributed in cells and tissues of the central nervous system (CNS). In particular, for treatment of diseases with CNS etiology, such enzyme replacement therapies have been problematic because the intravenously administered proteins and/or enzymes do not pass the blood-brain barrier (BBB).

In order to overcome these problems with the blood-brain barrier, attempts have been made to develop a treatment method in which proteins and/or enzymes are directly administered into the subject's cerebrospinal fluid by intrathecal (IT) injection; however, such attempts have not yet been successful in terms of stability and effectiveness.

Accordingly, there is a need for the development of a treatment method that can effectively treat Hunter syndrome patients with a central nervous disorder and ultimately bring about improved brain function in these patients.

The present inventors have found that in a case where iduronate-2-sulfatase beta is repeatedly administered into the cerebral lateral ventricle of a subject for a long time, such administration has a surprising effect of suppressing and improving brain function decline observed in patients with Hunter syndrome; and thus have completed the present invention.

### Technical Problem

An object of the present invention is to provide a therapeutic method and a pharmaceutical composition for substantially treating or improving central nervous system degeneration, in particular, brain function decline, caused by Hunter syndrome.

### Solution to Problem

In order to achieve the above object, the present invention provides a pharmaceutical composition for treating Hunter syndrome in a subject, which comprises iduronate-2-sulfatase beta and is administered once every 4 weeks into the cerebral lateral ventricle, and a method for treating Hunter syndrome using the same.

### Advantageous Effects of Invention

The pharmaceutical composition according to an aspect of the present invention exhibits effects such as actually improving brain function in a subject suffering from Hunter syndrome. In the present invention, it has been identified that administration of such a composition can sufficiently suppress brain function loss even in clinical practice.

In addition, the pharmaceutical composition according to the present invention, which is repeatedly administered over a long period of time, has a better therapeutic effect on Hunter syndrome than a case where the same dose of the active ingredient is given by single administration, and further has effects, which cannot be anticipated from the results obtained by the single administration, such as treating or restoring damaged brain structures, and substantially treating or improving brain function, in particular, improving memory and learning function.

### Brief Description of Drawings

FIG. 1 illustrates a diagram showing the location where a formulation according to an aspect of the present invention is administered in Test Example 1, in which the indicated location refers to the mouse cerebral lateral ventricle.
FIG. 2 illustrates pictures of extracting cerebrospinal fluid from the mouse brain in Test Example 2, in which the rightmost picture shows the cerebrospinal fluid collected in a micropipette.
FIG. 3 illustrates graphs, which are the results of Test Example 2, showing amounts of heparan sulfate (HS) accumulated in the brain and the cerebrospinal fluid of mice.
FIG. 4 illustrates graphs, which are the results of Test Example 2, showing comparison of amounts of HS accumulated in the brain and the cerebrospinal fluid of mice at a time point which is 28 days after single administration, and a time point which is 28 days after 6 repeated administrations made according to the Example.
FIG. 5A illustrates pictures obtained by brain magnetic resonance imaging of mice which are the results of Test Example 3; and FIG. 5B illustrates a graph showing proportions of cerebral ventricle volume relative to total brain volume in the mice.
FIG. 6 illustrates pictures, which are the results of Test Example 4, showing results obtained by performing hematoxylin and eosin staining of mouse brain tissues.
FIG. 7 illustrates pictures, which are the results of Test Example 5, showing results obtained by performing immunohistochemical staining of mouse brain tissues.
FIG. 8 illustrates graphs, which are the results of Test Example 6, showing results obtained by performing a fear conditioning test for evaluating learning-memory ability of mice.
FIG. 9 illustrates graphs, which are the results of Test Example 7, showing results obtained by performing an open field test for evaluating hyperactivity characteristics of mice.

### Detailed Description of Invention

### Definition

In order to better understand the present invention, certain terms are defined as follows. Further explanations for the terms as indicated below and other terms are set forth throughout the present specification.

As used herein, the term "about," as applied to one or more numerical values, refers to a numerical value that is similar to a stated reference numerical value. In an aspect of the present invention, the term "about" refers to a range of numerical values that fall within 25%, 20%, 19%, 18%, 17%, 16%, 15%, 14%, 13%, 12%, 11%, 10%, 9%, 8%, 7%, 6%, 5%, 4%, 3%, 2%, 1%, or less in either direction (greater than or lower than) of the stated numerical value unless otherwise stated or the context clearly indicates otherwise (except a case where such number exceeds 100% of a possible numerical value).

As used herein, the term "treatment" refers to any administration of a therapeutic protein (for example, lysosomal enzyme) that partially or completely alleviates, ameliorates, relieves, suppresses, delays onset of, decreases severity of and/or decreases incidence of one or more symptoms or features of a particular disease, disorder, and/or condition (for examples, Hunters syndrome). Such treatment may be applied to a subject who does not exhibit signs of the relevant disease, disorder, and/or condition, and/or to a subject who exhibits only early signs of the disease, disorder, and/or condition. Optionally or additionally, such treatment may be applied to a subject who exhibits one or more established signs of the relevant disease, disorder, and/or condition.

Hereinafter, the present invention will be described in detail.

In an aspect, the present invention may be directed to a pharmaceutical composition comprising iduronate-2-sulfatase beta ("IDS-β").

In an aspect of the present invention, the pharmaceutical composition may be for treating Hunter syndrome in a subject.

In an aspect of the present invention, the pharmaceutical composition may be for treating or inhibiting ventriculomegaly in a subject suffering from Hunter syndrome.

In an aspect of the present invention, the pharmaceutical composition may be for improving brain function in a subject suffering from Hunter syndrome.

In an aspect of the present invention, the IDS-β may include a protein having the amino acid sequence of SEQ ID NO: 1. SEQ ID NO: 1 is a recombinant human iduronate-2-sulfatase protein. In an aspect of the present invention, the IDS-β may further include a protein having the amino acid sequence of SEQ ID NO: 2. SEQ ID NO: 2 is a recombinant human iduronate-2-sulfatase protein in which cysteine at amino acid position 59 is substituted with formylglycine (Fgly).

In an aspect of the present invention, the IDS-β may include equal to or lower than about 20 to 35 mol% of a protein having the amino acid sequence of SEQ ID NO: 1 and equal to or lower than about 65 to 80 mol% of a protein having the amino acid sequence of SEQ ID NO: 2. Specifically, In an aspect of the present invention, the IDS-β may include equal to or lower than about 35 mol% of a protein having the amino acid sequence of SEQ ID NO: 1 and equal to or lower than about 65 mol% of a protein having the amino acid sequence of SEQ ID NO: 2.

In an aspect of the present invention, the IDS-β may include a protein having an amino acid sequence that is at least 60%, 65%, 70%, 75%, 80%, 85%, 90%, 95%, or 98% identical to SEQ ID NO: 1. In an aspect of the present invention, the IDS-β may include a protein having an amino acid sequence that is at least 60%, 65%, 70%, 75%, 80%, 85%, 90%, 95%, or 98% identical to SEQ ID NO: 2.

In an aspect of the present invention, the IDS-β may be at a concentration of about 0.1 mg/ml to about 60 mg/ml, about 1 mg/ml to about 10 mg/ml, or about 6 mg/ml.

In an aspect of the present invention, a dose of the IDS-β at the time of single administration may be about 1 µg to 60 µg, about 15 µg to about 60 µg, about 20 µg to about 40 µg, about 25 µg to about 35 µg, about 29 µg to about 31 µg, or about 30 µg; and such a dose may be a dose in a case of being administered to a mammal such as a mouse. However, the present invention is not limited thereto. In addition, in an aspect of the present invention, a dose of the IDS-β at the time of single administration may be about 1 mg to 60 mg, about 15 mg to about 60 mg, about 20 mg to about 40 mg, about 25 mg to about 35 mg, about 29 mg to about 31 mg, or about 30 mg; and such a dose may be a dose in a case of being administered to a human. However, the present invention is not limited thereto.

In an aspect of the present invention, the pharmaceutical composition may be administered once every 4 weeks into the cerebral lateral ventricle of the subject.

In an aspect of the present invention, the pharmaceutical composition may be administered directly to either or both of the cerebral lateral ventricles of the subject.

In an aspect of the present invention, administration of the pharmaceutical composition may decrease or inhibit ventriculomegaly in a subject with Hunter syndrome; improve the subject's brain function; improve the subject's learning and memory ability; suppress the subject's hyperactivity characteristics; decrease cellular vacuolization in brain cells; or inhibit expression of lysosome-associated membrane protein 2 (LAMP2) in brain cells.

In an aspect of the present invention, administration of the pharmaceutical composition may decrease accumulation of heparan sulfate (HS) in the brain (for example, brain cells) or cerebrospinal fluid of the subject. In an aspect of the present invention, the HS accumulation may be decreased by 20%, 40%, 50%, 60%, 80%, 90%, 1-fold, 1.5-fold, 2-fold, or 3-fold as compared with a control.

In an aspect of the present invention, administration of the pharmaceutical composition may be performed three or more times to the subject. In an aspect of the present invention, administration of the pharmaceutical composition may be performed 3 or more times, 4 or more times, 5 or more times, 6 or more times, 7 or more times, 8 or more times, 9 or more times, or 10 or more times; and may specifically be performed 6 times to the subject.

In an aspect of the present invention, administration of the pharmaceutical composition may be performed for 12 weeks or longer and 48 weeks or shorter. In an aspect of the present invention, administration of the pharmaceutical composition may be performed for 12 weeks or longer, 16 weeks or longer, 20 weeks or longer, 24 weeks or longer, 28 weeks or longer, 32 weeks or longer, 36 weeks or longer, 40 weeks or longer, 44 weeks or longer, or 48 weeks or longer; and may specifically be performed for 24 weeks.

In an aspect of the present invention, the subject may be a human or mammal. In an aspect of the present invention, the mammal may be a mouse, rat, rodent, cat, rabbit, dog, pig, horse, or cow.

In an aspect of the present invention, administration of the pharmaceutical composition may be performed through an intraventricular catheter system including a reservoir and a catheter connected to the reservoir. In the test examples as described later, since the administration was made to mice, separate three-dimensional positioning device and three-dimensional positioning coordinate system were required. However, in clinical application, the pharmaceutical composition of the present invention can be administered through a simple intraventricular catheter system.

In an aspect of the present invention, administration of the pharmaceutical composition may be performed with a method comprising steps of: 1) surgically inserting the catheter system, in which the reservoir is located under the scalp of the subject and an end of the catheter is located in the cerebral lateral ventricle of the subject, so that a cerebral lateral ventricular inner space is connected, via a catheter inner space, to a reservoir inner space, thereby allowing cerebrospinal fluid to flow from the cerebral lateral ventricle to the reservoir and causing the reservoir to be filled with the cerebrospinal fluid; 2) withdrawing about 0.1 to 5 ml of the cerebrospinal fluid from the reservoir at a rate of 0.1 to 60 ml/min; 3) injecting 0.1 to 5 ml of the pharmaceutical composition into the reservoir at a rate of 0.1 to 60 ml/min; and 4) allowing the pharmaceutical composition to flow from the reservoir, via the catheter, to the cerebral lateral ventricle.

In an aspect of the present invention, administration of the pharmaceutical composition may be performed in combination with one or more additional enzyme replacement therapies for treating Hunter syndrome. Specifically, in an aspect of the present invention, the additional enzyme replacement therapy may be at least one of intravenous administration and subcutaneous administration of IDS-β. Such intravenous administration and subcutaneous administration may refer to conventional administration methods; and in clinical application, cerebral lateral ventricular administration of the present invention does not require setting a limit for the administration schedule.

In an aspect of the present invention, the pharmaceutical composition may further include sodium chloride and polysorbate 20, and may have a pH of about 5 to about 7. In an aspect of the present invention, the pharmaceutical composition may have a pH of about 6.

In an aspect of the present invention, sodium chloride contained in the pharmaceutical composition may have a concentration of about 100 mM to about 200 mM Here, the concentration of sodium chloride may correspond to a range of all integer values that exist within the above-mentioned range. Specifically, sodium chloride may have a concentration of about 150 mM.

In an aspect of the present invention, polysorbate 20 contained in the pharmaceutical composition may have a concentration of about 0.01 mg/ml to 0.5 mg/ml. Here, the concentration of polysorbate 20 may correspond to a range of all decimal values that exist within the above-mentioned range. Specifically, polysorbate 20 may have a concentration of about 0.05 mg/ml. In an aspect of the present invention, administration of the pharmaceutical composition may not cause any severe adverse effects. In an aspect of the present invention, the severe adverse effect may refer to, but is not limited to, any effect that causes substantial immune response, toxicity, or death.

As used herein, the term "substantial immune responses" may refer to severe or serious immune responses, such as substantial adaptive T-cell mediated immune response.

In an aspect of the present invention, the pharmaceutical composition may be provided in a form filled into a container, such as a vial, a syringe, or a pre-filled syringe, and may be provided in a form filled into a disposable plastic syringe in a case of being administered in a single dose. In an aspect of the present invention, the pharmaceutical composition may be administered in a volume of about 1 µL to about 10 µL, a volume of about 3 µL to about 7 µL, a volume of about 4 µL to about 6 µL, or a volume of about 5 µL; however, the present invention is not limited thereto. In addition, in an aspect of the present invention, the pharmaceutical composition may be administered in a volume of about 1 mL to about 10 mL, a volume of about 3 mL to about 7 mL, a volume of about 4 mL to about 6 mL, or a volume of about 5 mL; however, the present invention is not limited thereto.

In an aspect, the present invention may be directed to a pharmaceutical composition for treating or inhibiting ventriculomegaly in a subject suffering from Hunter syndrome, comprising about 150 mM sodium chloride, about 0.05 mg/ml of polysorbate 20, and about 5 mg/ml of iduronate-2-sulfatase beta (IDS-β), in which the pharmaceutical composition has a pH of about 6 and is administered once every 4 weeks into the cerebral lateral ventricle.

In an aspect, the present invention may be directed to a pharmaceutical composition for improving brain function in a subject suffering from Hunter syndrome, comprising about 150 mM sodium chloride, about 0.05 mg/ml of polysorbate 20, and about 5 mg/ml of iduronate-2-sulfatase beta (IDS-β), in which the pharmaceutical composition has a pH of about 6 and is administered once every 4 weeks into the cerebral lateral ventricle.

In an aspect, the present invention provides a method for treating Hunter syndrome, comprising a step of applying to a subject the pharmaceutical composition of the present invention.

In an aspect of the present invention, there is provided a use of the pharmaceutical composition of the present invention for treating Hunter syndrome.

In an aspect of the present invention, there is provided a use of the pharmaceutical composition of the present invention for manufacture of a medicament for treating Hunter syndrome.

Hereinafter, the configuration and effects of the present specification will be described in more detail by way of examples and test examples. However, these examples and test examples are provided for illustrative purposes only to help understand the present specification, and the scope of the present specification is not limited by the following examples.

### [Preparation Example 1] Preparation of IDS-β formulation for administration

As a carrier solution, 150 mM sodium chloride and 0.05 mg/ml of Tween 20 solution (Merck Millipore, Darmstadt, Germany) were used. An IDS-β solution (50 mg/ml or 15 mg/ml) obtained from Green Cross Co., Ltd. was diluted in the above carrier solution, to prepare an IDS-β formulation at a concentration of 6 mg/ml. The thus prepared IDS-β formulation was used in the test examples below.

### [Test Example 1] Cerebral lateral ventricular administration to mice

### Animal

The mice used in the test examples as described in the present specification were in-house-produced mice, and were produced in such a manner that a part of iduronate 2-sulfatase (IDS) gene was deleted. Briefly, deletion in the IDS gene was made from exons 2 to 3. IDS KO mice (IDS knock-out mice) were bred from a C57BL/6.129S background strain and had a null mutation in the IDS gene. Wild-type (WT) control mice were bred from a C57BL/B6.129S strain. The genotypes of all mice were checked by polymerase chain reaction (PCR) using DNA obtained from a tail snip. The test examples in the present specification were approved by the Institutional Animal Care and Use Committee (Approval No. 20140925005) and conducted in accordance with the animal welfare policy of the Samsung Biomedical Research Institute (Seoul, Korea).

The mice obtained by breeding were classified and assigned as shown in Table 1 below.

**[Table 1]**

| Group | Type of mouse | Dosage | Number of administration | Number of subjects |
|---|---|---|---|---|
| Reference Example | Wild-type control mouse | Carrier (untreated) | 6 | 12 |
| Comparative Example 1 | IDS KO mouse | Carrier (untreated) | 6 | 12 |
| Comparative Example 2 | IDS KO mouse | 30 µg of IDS-β, single administration | 1 | 12 |
| Example | IDS KO mouse | Total 180 µg of IDS-β, administered once every 4 weeks at a single dose of 30 µg | 6 | 12 |

### Cerebral lateral ventricular administration

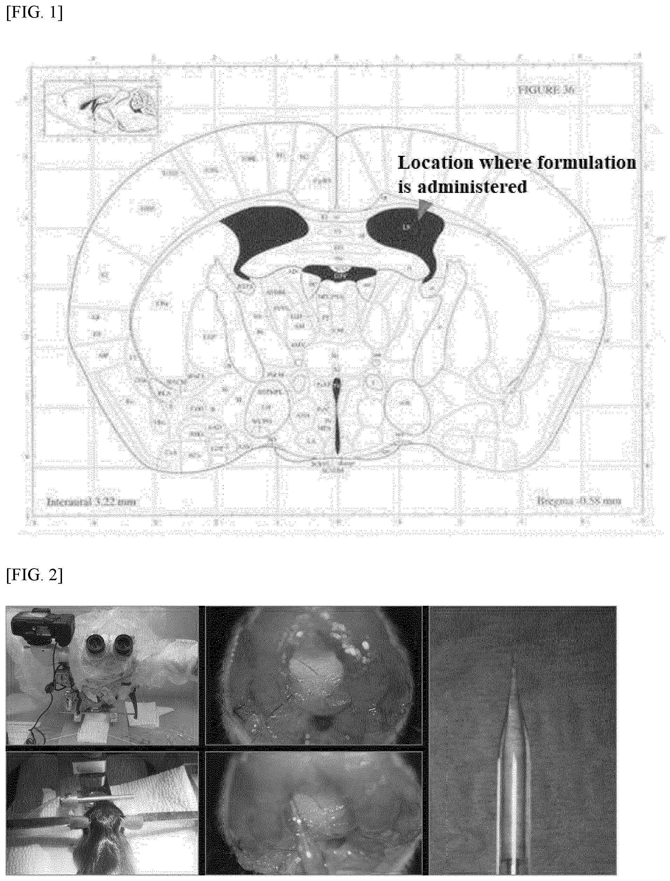

The mouse brain coordinates are required for accurate cerebral lateral ventricular administration. This information is described in [Paxinos, George and Franklin, Keith B. J., Paxinos and Franklin's The Mouse Brain in Stereotaxic Coordinates, Academic Press, 4th Edition, 2012] and is as illustrated in FIG. 1. For the location of the cerebral lateral ventricle with mouse age based on weeks, reference was further made to [Paxinos, George and Franklin, Keith B. J., Paxinos and Franklin's The Mouse Brain in Stereotaxic Coordinates, Academic Press, 4th Edition, 2012].

Intracerebral lateral ventricular administration was performed for 6-week-old mice obtained by the above breeding. Each group was administered the carrier solution and the IDS-β solution of Preparation Example 1 in a volume of 5 µL at the time of single administration. On the day of administration, the mice were anesthetized with isoflurane (HANA PHARM CO., LTD., Korea) by inhalation and placed in a stereotactic instrument (Stoelting, USA). Then, the scalp of the mouse was minimally peeled off, and the exposed skull was cleaned. Each of the carrier solution and IDS-β solution prepared in Preparation Example 1 was placed in a disposable plastic syringe, and injected into the cerebral lateral ventricle of the mouse with a 31-gauge needle at a constant rate of 10 µL/min using a syringe pump (KD Scientific, Switzerland). During administration, the position of the syringe needle in the brain was monitored using the stereotaxic coordinates (Stoelting, USA). The coordinates of the needle at the start of administration were as follows: -0.58 mm caudal to bregma, 1.25 mm lateral to sagittal suture, and -1.77 mm in depth. After completing administration of the volume of 5 µL, an act of lifting the syringe needle by 0.15 mm per 30 seconds for removal thereof from the injection site was repeated 4 to 5 times. Then, an act of lifting the needle at a height of 0.3 mm per 30 seconds from the -1.00 mm in depth position was repeated several times to completely remove the syringe needle. These acts were introduced to prevent backflow of the administered solution, and the time required for the entire administration process was about 30 minutes per subject. After the administration, the exposed skin area including the injection site was simply sutured with a medical stapler.

After this administration, the mice did not show any serious adverse effects. No subject dropout, which is due to substantial immune response at the administration site, abnormal body function, or the like, was observed. Autopsy was performed 28 days after the last administration date so that the following analysis was conducted.

### [Test Example 2] Analysis of HS accumulated in brain or cerebrospinal fluid of mouse

### Collection of brain and cerebrospinal fluid of mouse

At the time of autopsy, which is 28 days after the last administration date, for each group according to Table 1, the cerebrospinal fluid was first collected using a borosilicate glass micropipette (outer diameter: 10 mm, inner diameter: 0.75 mm) at the cisterna magna area of the mouse of interest (see FIG. 2). For HS analysis, the collected cerebrospinal fluid (approximately 5 to 10 µL) was rapidly frozen using liquid nitrogen immediately after collection, and then stored in a cryogenic freezer until the analysis. For analysis of heparan sulfate in the brain, transcardial perfusion was performed with phosphate buffered saline (PBS), and the brain was collected. The collected brain was rapidly frozen, and then stored in a cryogenic freezer until the analysis.

### HS analysis method

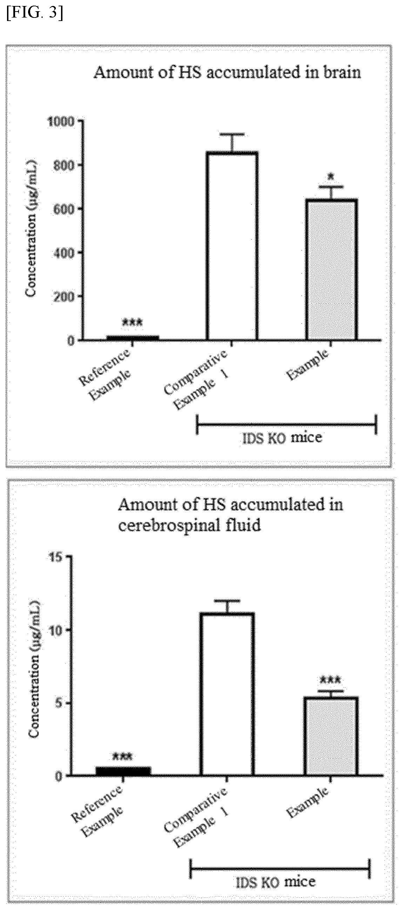

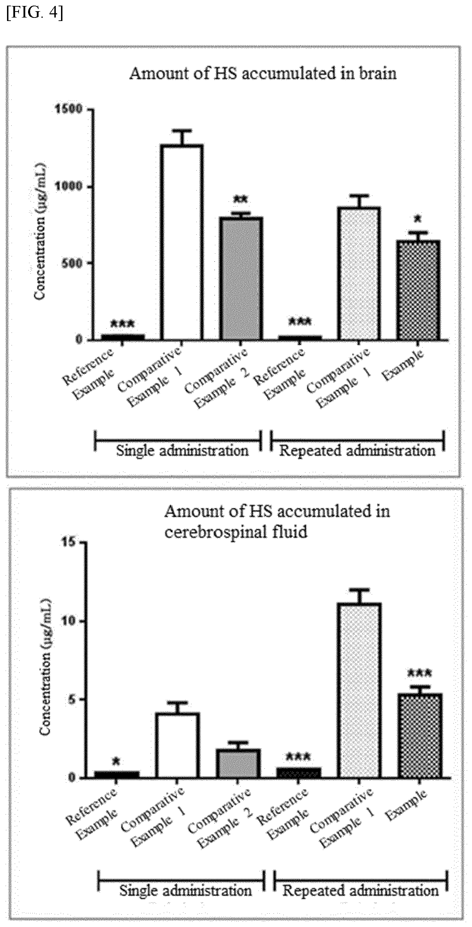

HS levels in the cerebrospinal fluid and brain tissue samples collected from mouse were measured using liquid chromatography tandem-mass spectrometry (LC-MS/MS). A 2 µL sample of the mouse cerebrospinal fluid or brain tissue, which had been homogenized in PBS, was aliquoted for pretreatment. Each of the samples was heated in hydrochloric acid-methanol to be mathanolyzed. The resulting methanolysate was evaporated to dryness under a stream of nitrogen gas. As internal standards, deuterated dermatan sulfate and heparan sulfate (DS-d6 and HS-d6) were used. The thus obtained solution was transferred to a centrifugal filter and centrifugation was performed. The resulting filtrate was analyzed. LC-MS/MS analysis was performed using an ultra performance liquid chromatography system (UPLC, Waters) and a triple quadrupole mass spectrometer (API5000, AB/MDS Sciex). Data were processed using the Analyst 1.5.1. software. The analysis results are illustrated in FIGS. 3 and 4, and statistical analysis was performed by the multiple comparison test of Holm-Sidak (in FIGS. 3 and 4, *: P<0.05, **: P<0.001, ***: P<0.0001).

### Results

Amounts of HS accumulated in the brain and the cerebrospinal fluid in the respective groups were compared. Referring to FIG. 3, it was identified that for the Example of the present invention, at a time point which is 28 days after the last administration date, HS was maintained at a lower level than that of Comparative Example 1. These results suggest that the once every 4 weeks administration cycle exhibits an effect of inhibiting HS accumulation from the viewpoint that the effect has lasted steadily for one month at the administered dose (see FIG. 3).

Referring to FIG. 4, it was identified that in a case of comparing amounts decreased of HS between the mice of Comparative Example 2, which had been administered the same amount of the drug at the time of single administration, and the mice of the Example of the present invention, more HS was decreased in the Example, in which repeated administration had been made, than in Comparative Example 2, in which single administration had been made. These results show that continuous administration results in much better therapeutic effects than single administration even if the single administration is made in such a manner that the same single dose is administered and the same period of time (that is, 28 days after administration) is maintained; and this has great implications in clinical therapeutic effects. According to these results, in a case where the same single dose is used, repeated administration will result in remarkably increased therapeutic effects in patients; and convenience of patients and caregivers must be supported for this.

### [Test Example 3] Evaluation of inhibitory effect on ventriculomegaly

Brain MRI analysis of the mice was performed to identify whether the cerebral lateral ventricular administration according to the present invention not only improves HS, which is a biochemical indicator of brain and cerebrospinal fluid, but also has an effect of inhibiting structural destruction or damage of the brain.

Specifically, after completion of 6 administrations, all mouse images were obtained using a 7.0-Tesla MRI system (Bruker-Biospin, Fallanden, Switzerland), and this system was equipped with a 20-cm gradient set capable of supplying up to 400 mT/m with a rise time of 100 ms. A birdcage coil (inside diameter, 72 mm; Bruker Biospin) was used for excitation, and an actively decoupled phased array coil was used for signal reception.

The mice were anesthetized with isoflurane (HANAPHARM CO., LTD., Korea) (5% for induction, 2% for the animal setup, and 1% to 1.5% during the MRI experiment) in a mixture of oxygen and nitrogen gases (3:7) delivered to a nose cone for spontaneous respiration throughout the experiment. The head of the mouse was very carefully fixed using a bite/ear bar. Coronal images containing the cerebellum were obtained with the following characteristics: T2-weighted, fast spin-echo sequence with 192 × 192 matrix size, 19.2 × 19.2 mm² resolution, TR/TE = 3,000/70 ms, NEX = 6, 1.0 mm slice thickness with no interslice gap.

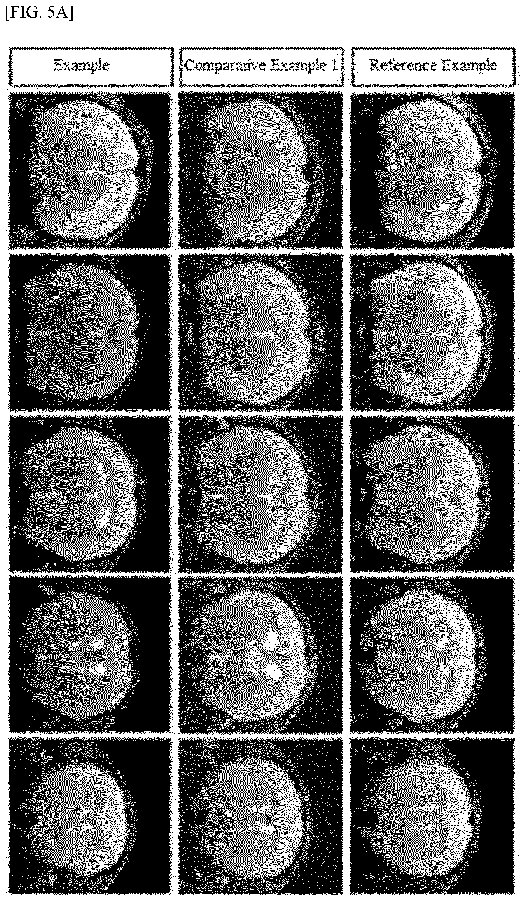

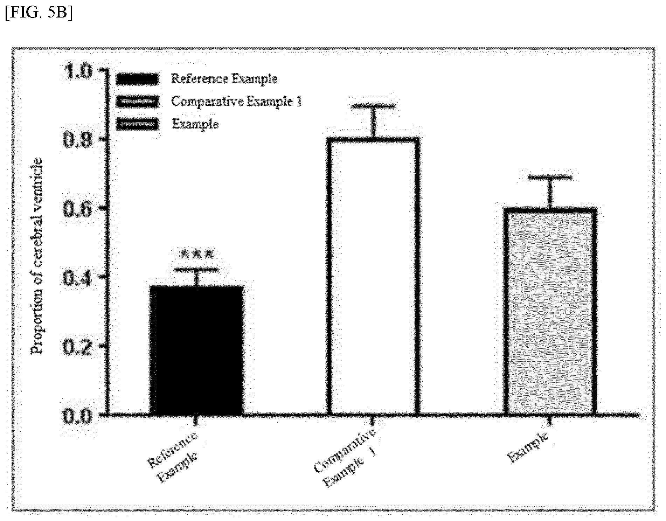

To assess the percent volume ratio of ventricles relative to whole brain, 9 coronal MRIs per mouse were obtained. The percent volume ratio was manually estimated by outlining serial images in a blinded manner using Para Vision 2.0.2 software (Bruker, BioSpin, Karlsruhe, Germany). The boundaries of whole brain and ventricle were outlined on each image, and the corresponding area was calculated using the above-mentioned software. The cerebral ventricle area was calculated as (lateral ventricle area/total brain area). These results are shown in FIG. 5 and Table 2.

### Results

Among Hunter syndrome patients, those with central nervous system degeneration were accompanied by ventriculomegaly. Similarly, even in Hunter syndrome mice (IDS KO mice), ventriculomegaly could be identified by brain MRI. In this test example, it was verified whether among the IDS KO mice, ventriculomegaly in the untreated group is alleviated by the administration of the present invention. As a result, it was found that the Example exhibited significantly decreased ventricles as compared with the untreated group, Comparative Example 1.

**[Table 2]**

| Group | Dosage | Proportion of ventricles in brain (%) (mean ± SEM) |
|---|---|---|
| Reference Example | Carrier (untreated) | 0.37 ± 0.06 |
| Comparative Example 1 | Carrier (untreated) | 0.80 ± 0.10 |
| Example | Total 180 µg of IDS-β, administered once every 4 weeks at a single dose of 30 µg | 0.60 ± 0.10 |

### [Test Example 4] Evaluation of inhibitory effect on cellular vacuolization

At the time of autopsy of the mice in each group, among mouse brain tissues, sections of the cerebellum, medulla, thalamus, and cortex were collected for tissue staining, fixed with a 4% paraformaldehyde solution (BIOSESANG, Korea) overnight, subjected to tissue processing, and then embedded in paraffin. 4 µm-thick sections were prepared for hematoxylin/eosin (H&E) staining.

### Hematoxylin/eosin staining

Hematoxylin/eosin staining was performed on the brain tissue sections collected from the mice in each group.

Specifically, a slide, on which the brain tissue section was fixed, was immersed in a xylene solution to perform deparaffinization of the slide. Then, the slide was immersed sequentially in ethanol ranging from 100% ethanol to lower-concentration ethanol, to remove xylene. Subsequently, the slide was washed with running water, and then stained by being immersed in a hematoxylin solution for 5 to 8 minutes. After staining, the slide was washed with running water, immersed in 1% hydrochloric acid for a while, and then washed again with running water. The slide was immersed in a 1% ammonia solution, and then washed again with running water. Subsequently, the slide was stained by being immersed in an eosin solution for 2 minutes. Then, the slide was dehydrated with an ethanol solution, and sealed with xylene.

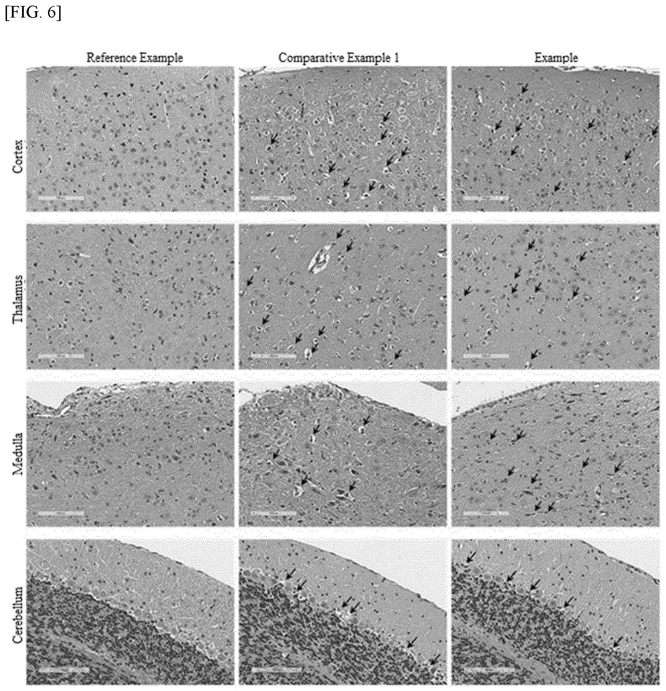

The stained slide was scanned with the ScanScope AT (Aperio, Leica, German) device, and then analyzed with the ImageScope (Aperio, Leica, German) device to evaluate histopathological morphology. The pictures for the scanned slides are illustrated in FIG. 6.

### Results

The hematoxylin/eosin staining showed that for Hunter syndrome mice, structural destruction was ongoing throughout the brain due to accumulation of glycosaminoglycans (GAGs). Specifically, for the mice with Hunter syndrome in Comparative Example 1, cellular vacuolization, which is a typical marker of lysosomal storage diseases, was found throughout the mouse brain, as compared with the mice in Reference Example (see FIG. 6). This cellular vacuolization is indicated by an arrow in the pictures of the stained slides for Reference Example 1 in FIG. 6. On the other hand, it was found that for the mice with Hunter syndrome in the Example, after the administration according to the present invention, cellular vacuolization was extensively decreased in the areas indicated by an arrow.

### [Test Example 5] Evaluation of inhibitory effect on expression of LAMP2 protein

### Immunohistochemical staining

The brain tissue sections of the respective groups prepared in Test Example 4 were stained with anti-LAMP-2 IgG (Santa Cruz Biotechnology, USA), which is a primary antibody, and were developed by the DAB method to visualize the expression of LAMP2 protein. The LAMP2 protein is a lysosome-associated membrane protein and is used as an indicator of lysosome activity. After being developed by the DAB method, the brain tissue sections were counter-stained with hematoxylin for the LAMP2 protein-positive cells and nuclei.

Specifically, the 4-µm thick tissue sections were allowed to stand in 10% normal goat serum (Dako, Carpinteria, CA, USA) for 20 minutes at room temperature, and then allowed to stand at room temperature for 30 minutes together with anti-LAMP-2 antibody in a ratio of 1:100. The tissue sections were washed several times with PBS. Then, the tissue sections were allowed to stand for 30 minutes at room temperature together with HRP-labeled polymer-conjugated secondary antibody. The tissue sections were allowed to stand in a 3,3-diaminobenzidine substrate-chromogen solution (Dako, Carpinteria, CA, USA) for 5 minutes, and washed with water. Then, nuclear staining was performed with hematoxylin.

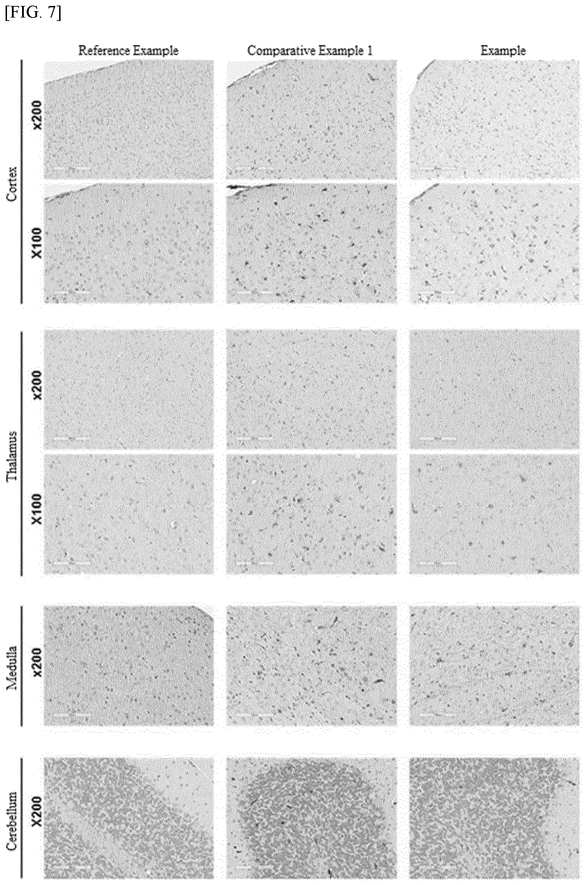

The stained slide was scanned with the ScanScope AT (Aperio, Leica, Germany) device, and then analyzed with the ImageScope (Aperio, Leica, Germany) device to evaluate histopathological morphology. The pictures for the scanned slides are illustrated in FIG. 7.

### Results

Referring to FIG. 7, it was found that the mice with Hunter syndrome in Comparative Example 1 exhibited a remarkably high expression level of LAMP2 protein, which is a marker of lysosome activity and disease status in brain cells and perivascular cells, as compared with the mice in the Reference Example. On the other hand, it was found that after the administration according to the present invention, the mice with Hunter syndrome in the Example exhibited a remarkably inhibited expression level of LAMP2 protein throughout the brain tissue.

### [Test Examples 6 and 7] Mouse behavioral assessment for evaluation of brain function improvement

In order to identify an actual effect of the administration of the present invention on brain function, a fear conditioning test and an open field test were performed which are tests for evaluating learning-memory ability among animal behavioral assessments. These two behavioral assessment items can be used to evaluate overall brain function by evaluating the exploration ability or movement of small animals such as rodents, and their degree of coping with sufficiently anticipated environmental changes. The methods for performing the respective tests and the test results are as follows.

### [Test Example 6] Fear conditioning test

At weeks 2 to 3 after the cerebral lateral ventricular administration of Test Example 1 (after the 1st, 3rd, and 6th administrations), fear memory of the mice belonging to each group was evaluated using the following fear conditioning system (Coulbourn Instruments): On day 1 of evaluation, the mice were stimulated in a fear conditioning chamber with a 30-second auditory cue (80 dB, 3600 Hz tone) as a conditioned stimulus and a 2-second foot shock (0.6 mA direct current) as an unconditioned stimulus. On day 2 of evaluation which is 24 hours later, the mice were placed back in the same chamber, and video-recorded at a frequency of 40 Hz (30 frames per 0.75 seconds) for 300 seconds to evaluate associative memory. 1 hour after the associative memory evaluation, in order to evaluate the cue memory of the mice, the mice were placed in a new and different chamber and given stimuli with an auditory cue. Mouse movements were likewise video-recorded for 300 seconds at a frequency of 40 Hz (30 frames per 0.75 seconds).

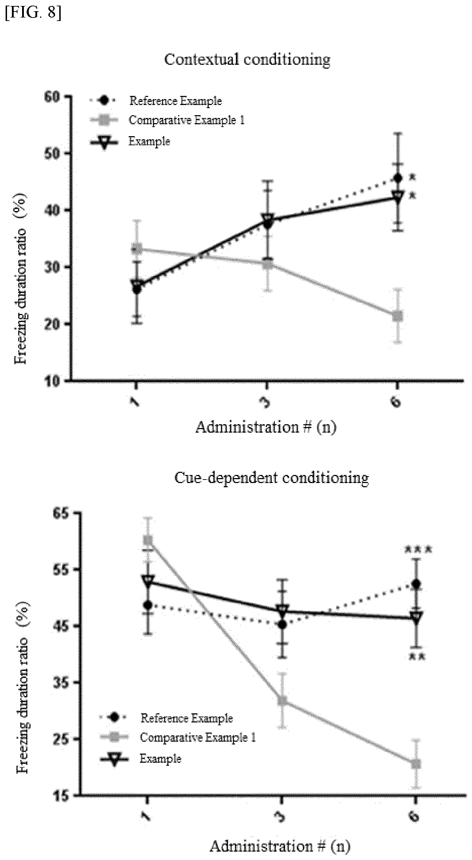

The recorded videos were analyzed post hoc using Freeze Frame Software Ver 3.32, which automatically calculated motion index and relative absence of motion. Freezing duration was counted when the mice displayed a bout of relative absence of motion for longer than 0.75 seconds or 2 seconds. This freezing duration was divided by total observation duration (300 seconds). The resulting value was expressed as a percentage, and the graphs for contextual conditioning and cue-dependent conditioning items are illustrated in FIG. 8. The specific results are shown in Table 3.

**[Table 3]**

| Group | Administration # | Contextual conditioning freezing duration ratio (%) (mean ± SEM) | Cue-dependent conditioning freezing duration ratio (%) (mean ± SEM) |
|---|---|---|---|
| Reference Example | 1 | 26.25 ± 4.80 | 48.84 ± 5.15 |
| | 3 | 37.62 ± 5.92 | 45.40 ± 5.87 |
| | 6 | 45.77 ± 7.86 | 52.61 ± 4.34 |
| Comparative Example 1 | 1 | 33.32 ± 5.01 | 60.31 ± 3.89 |
| | 3 | 30.72 ± 4.79 | 31.89 ± 4.74 |
| | 6 | 21.53 ± 4.62 | 20.70 ± 4.20 |
| Example | 1 | 26.72 ± 6.48 | 52.93 ± 5.59 |
| | 3 | 38.36 ± 6.86 | 47.69 ± 5.67 |
| | 6 | 42.35 ± 5.84 | 46.48 ± 5.18 |

### Results

Referring to FIG. 8, it is identifiable that as the administration proceeds, the mice in the Example exhibited remarkably superior learning-memory ability as compared with the mice in Comparative Example 1. After the final 6th administration, the mice in the Example had improved brain function to the extent that they exhibited learning-memory ability close to that of a normal mouse. For both the 'contextual conditioning' item, in which associative memory (for evaluation of visual and auditory memory) is measured in the test space, and the 'cue-dependent conditioning' item, in which fear memory for repeated fear stimuli is evaluated, it can be seen that clear improvement is observed starting from a time point of the 3rd administration (see FIG. 8 and Table 3).

### [Test Example 7] Open field test

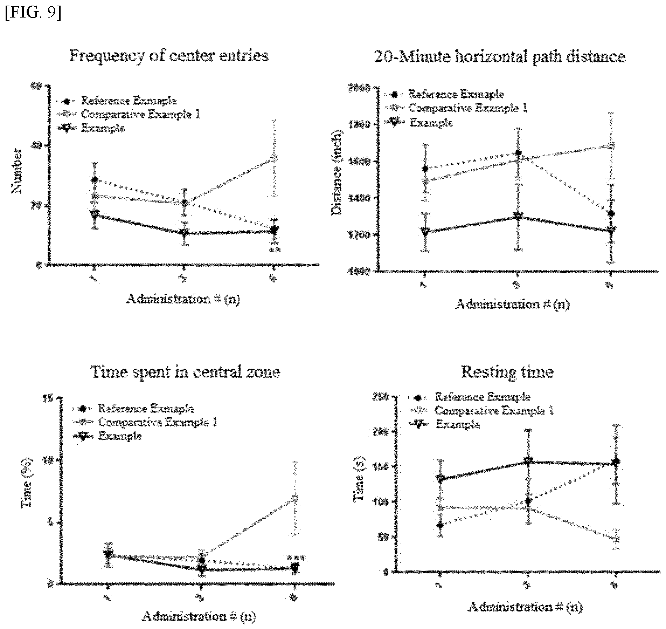

After the cerebral lateral ventricular administration of Test Example 1 (after the 1st, 3rd, and 6th administration), the following open field test was used to evaluate exploratory behavior of the mice in each group: The mice were allowed to explore an open-top arena (44.5 cm × 44.5 cm) sided with opaque walls (15.0 cm height) and their movement was video-recorded for 20 minutes. Recorded videos were analyzed for exploration distance, moving speed, resting time, time spent in the central and peripheral zones, and frequency of entry into each zone. The central zone means an area of 31.0 cm × 31.0 cm marked in the center of the arena, and the peripheral zone means the rest of the arena excluding the central zone. The graphs for the respective analysis results are illustrated in FIG. 9 and the specific result values are shown in Table 4.

### Results

It is known that Hunter syndrome mice usually exhibit hyperactivity characteristics. This was also identified in the open field test results of the present invention. 20-minute observation shows that the sum of distances traveled over the entire section (that is, horizontal path distance) steadily increased in the mice in Comparative Example 1. However, it was found that for the mice in the Example, the sum of distances traveled decreased, indicating suppression of hyperactivity characteristics. In the same sense, the mice in Comparative Example 1 also showed a decreasing trend of resting time, during which no movement is made, whereas the mice in the Example showed an increased resting time.

In certain space, rodents have an instinct to prefer sides which are easier to protect them from predators such as birds rather than the center. Therefore, in the open field test, it is a general behavioral characteristic of mice to explore the peripheral zone rather than the central zone in a prepared space. However, it was found that the mice in Comparative Example 1 showed increased frequency of center entries and time spent in the central zone with aging. On the other hand, the mice in the Example showed a similar tendency to the mice in the Reference Example which are normal mice.

**[Table 4]**

| Group | 20-minute horizontal path distance (inch) | | | Resting time (s) | | |
|---|---|---|---|---|---|---|
| | 1 | 3 | 6 | 1 | 3 | 6 |
| Reference Example | 1562.89±128.8 | 1646.75±133.3 | 1318.58 ± 156.7 | 67.51 ± 15.7 | 101.64 ± 31.8 | 8159.23 ± 32.8 |
| Comparative Example 1 | 1494.23 ± 109.2 | 1609.97 ± 107.5 | 1686.18 ± 180.1 | 92.27 ± 23.3 | 91.49 ± 21.7 | 47.24 ± 14.3 |
| Example | 1216.45 ± 101.2 | 1298.97 ± 177.7 | 1221.78 ± 170.2 | 132.66 ± 27.1 | 157.11 ± 45.6 | 153.76 ± 55.9 |

| Group | Frequency of center entries (n) | | | Time spent in central zone (%) | | |
|---|---|---|---|---|---|---|
| | 1 | 3 | 6 | 1 | 3 | 6 |
| Reference Example | 28.69±5.6 | 21.13 ± 4.3 | 12.25 ± 3.2 | 2.34 ± 0.6 | 1.91 ± 0.6 | 1.29 ± 0.4 |
| Comparative Example 1 | 23.29 ± 4.6 | 20.71 ± 3.3 | 35.86 ± 12.7 | 2.26 ± 0.6 | 2.20 ± 0.6 | 6.95 ± 2.9 |
| Example | 16.87 ± 44 | 10.67 ± 38 | 11.40 ± 3.9 | 2.39 ± 0.9 | 1.17 ± 0.5 | 1.29 ± 0.4 |

## Claims

1. A pharmaceutical composition for decreasing or inhibiting ventriculomegaly in a subject with Hunter syndrome, improving brain function or learning-memory ability of a subject with Hunter syndrome, or suppressing hyperactivity characteristics of a subject with Hunter syndrome, the pharmaceutical composition comprising:
iduronate-2-sulfatase beta (IDS-β),
wherein the pharmaceutical composition is administered once every 4 weeks into the cerebral lateral ventricle,
the administration does not cause any severe adverse effects in the subject, and
the administration decreases accumulation of heparan sulfate (HS) in brain cells or cerebrospinal fluid of the subject; decreases cellular vacuolization in brain cells of the subject; or inhibits expression of lysosome-associated membrane protein 2 (LAMP2) in brain cells of the subject.

2. The pharmaceutical composition of claim 1, wherein the administration is made directly to either or both of the cerebral lateral ventricles of the subject.

3. The pharmaceutical composition of claim 1, wherein the HS accumulation is decreased by 20%, 40%, 50%, 60%, 80%, 90%, 1-fold, 1.5-fold, or 2-fold as compared with a control.

4. The pharmaceutical composition of claim 1, wherein the administration is performed three or more times to the subject.

5. The pharmaceutical composition of claim 1, wherein the administration is made for 12 weeks or longer and 48 weeks or shorter.

6. The pharmaceutical composition of claim 1, wherein the subject is a mammal, human, or mouse.

7. The pharmaceutical composition of claim 6, wherein the subject is a mouse.

8. The pharmaceutical composition of claim 1, wherein the administration is performed through an intraventricular catheter system including a reservoir and a catheter connected to the reservoir.

9. The pharmaceutical composition of claim 8, wherein the administration is performed with a method that includes steps of:
1) surgically inserting the catheter system, in which the reservoir is located under the scalp of the subject and an end of the catheter is located in the cerebral ventricle of the subject, so that a cerebral ventricular inner space is connected, via a catheter inner space, to a reservoir inner space, thereby allowing cerebrospinal fluid to flow from the cerebral ventricle to the reservoir and causing the reservoir to be filled with the cerebrospinal fluid;
2) withdrawing about 0.1 to 5 ml of the cerebrospinal fluid from the reservoir at a rate of 0.1 to 60 ml/min;
3) injecting 0.1 to 5 ml of the pharmaceutical composition into the reservoir at a rate of 0.1 to 60 ml/min; and
4) allowing the pharmaceutical composition to flow from the reservoir, via the catheter, to the cerebral ventricle.

10. The pharmaceutical composition of claim 1, wherein the administration is performed in combination with one or more additional enzyme replacement therapies for treating Hunter syndrome.

11. The pharmaceutical composition of claim 10, wherein the additional enzyme replacement therapy is at least one of intravenous administration and subcutaneous administration of IDS-β.

12. The pharmaceutical composition of claim 1, wherein the IDS-β has a concentration of about 0.1 mg/ml to about 60 mg/ml, or about 1 mg/ml to about 10 mg/ml, or 6 mg/ml.

13. The pharmaceutical composition of claim 1, wherein a dose of the IDS-β at the time of single administration is about 15 µg to about 60 µg, or about 20 µg to about 40 µg, or about 30 µg.

14. The pharmaceutical composition of claim 1, wherein a dose of the IDS-β at the time of single administration is about 1 mg to about 60 mg, or about 15 mg to about 60 mg, or about 20 mg to about 40 mg, or about 30 mg.

15. The pharmaceutical composition of claim 1, further comprising:
sodium chloride; and
polysorbate 20,
wherein the pharmaceutical composition has a pH of 5 to 7.

16. The pharmaceutical composition of claim 15, wherein the sodium chloride has a concentration of about 100 mM to about 200 mM, and the polysorbate 20 has a concentration of about 0.01 mg/ml to 0.5 mg/ml.

17. The pharmaceutical composition of claim 1, wherein the severe adverse effect is a substantial adaptive T-cell mediated immune response, toxicity, or death.

18. A pharmaceutical composition for treating or inhibiting ventriculomegaly or improving brain function in a subject suffering from Hunter syndrome, comprising:
about 150 mM sodium chloride;
about 0.05 mg/ml of polysorbate 20; and
about 6 mg/ml of iduronate-2-sulfatase beta (IDS-β),
wherein the pharmaceutical composition has a pH of about 6 and is administered once every 4 weeks into the cerebral lateral ventricle.

19. A method for treating Hunter syndrome, comprising:
a step of applying to a subject the pharmaceutical composition of claim 1.

20. A use of the pharmaceutical composition of claim 1 for treating Hunter syndrome.

21. A use of the pharmaceutical composition of claim 1 for manufacture of a medicament for treating Hunter syndrome.
